# EUROPEAN PATENT SPECIFICATION

(11) **EP 2 654 683 B1**
(45) Date of publication and mention of the grant of the patent: **06.05.2015**
(21) Application number: 10805756.3
(22) Date of filing: 23.12.2010
(51) Int. Cl.: A61K 8/27, A61K 8/29, A61K 8/73, A61K 8/81, A61Q 11/00, A61K 8/02

(54) **FILMS AND COMPOSITIONS COMPRISING THE SAME**
FILME UND ZUSAMMENSETZUNGEN DAMIT
FILMS ET COMPOSITIONS LES COMPRENANT

(43) Date of publication of application: 30.10.2013
(73) Proprietor: Colgate-Palmolive Company, New York, NY 10022 (US)
(72) Inventor: MARTINETTI, Melissa, A., Bridgewater New Jersey 08807 (US); NESTA, Jason, Cedar Knolls New Jersey 07927 (US); LEIGH, Leonora, Piscataway New Jersey 08854 (US); BOYD, Thomas, Metuchen New Jersey 08840 (US)
(74) Representative: Jenkins, Peter David
(86) International application number: PCT/US2010/061962
(87) International publication number: WO 2012/087328

(56) References cited:
- WO-A1-2010/126742
- WO-A2-2010/138547
- WO-A2-2011/094497
- US-A1- 2007 148 213

## Description

### BACKGROUND

The incorporation of conventional films in aqueous oral care products generally results in premature dissolution of the films and undesirable clouding of the composition. Thus, there remains a need for orally acceptable films that are stable in aqueous compositions, and able to deliver an active ingredient to the oral cavity during use.

International Patent Application Publication No. 2010/126742 describes a cleaning composition that contains film flakes with beneficial ingredients. US Patent Application Publication No. 2007/148213 discloses an oral care, personal care or cleansing composition with a carrier comprising a functional material and at least two polymers. International Patent Application Publication No. 2010/138547 relates to polymer matrix films, compositions comprising the polymer matrix films, and methods of preparing and using the same.

### SUMMARY

In one aspect, the present invention provides a water-stable film according to claim 1. The present disclosure also provides a water-stable film, comprising: one or more cellulosic polymers present in an amount from about 10 to about 50% of the film's dry weight, polyvinyl acetate present in an amount from about 8 to about 25% of the film's dry weight; and colloidal particles. In some embodiments, the colloidal particles are metal particles.

Some embodiments provide a composition comprising one or more of the films described herein, and an orally acceptable aqueous carrier.

Also provided are methods of treating or preventing a disease or condition of the oral cavity comprising contacting an oral cavity surface with any one of the compositions described herein.

Methods of making the films are also provided.

### DETAILED DESCRIPTION

As used throughout, ranges are used as shorthand for describing each and every value that is within the range. Any value within the range can be selected as the terminus of the range.

In the event of a conflict in a definition in the present disclosure and that of a cited reference, the present disclosure controls.

As used herein, the term "cellulosic polymer" is meant to refer to cellulose and cellulose derivatives such as cellulose ester derivatives and cellulose ether derivatives.

As used herein, the term "aqueous" refers to a total water content of greater than 50%, by weight, wherein the composition is preferably not an emulsion.

As used herein, the term "water-stable film" refers to a film that remains substantially undissolved in a medium having a total water content of from about 50 to about 90%, by weight, for two years at 25°C.

As used herein, the term "substantially clear" when used in reference to oral care products and material shall mean translucent or transparent.

Some aspects of the present disclosure provide a water-stable film, comprising: one or more hydrophilic polymers present in an amount from about 10 to about 50% of the film's dry weight; a hydrophobic polymer present in an amount from about 8 to about 25% of the film's dry weight; and metal particles.

Some aspects of the present disclsosure provide a water-stable film, comprising: one or more cellulosic polymers present in an amount from about 10 to about 50% of the film's dry weight; polyvinyl acetate present in an amount from about 8 to about 25% of the film's dry weight; and metal particles.

In the water-stable films of the invention, the polyvinyl acetate is present at a concentration of from about 10 to about 20% of the film's dry weight. In some embodiments, the polyvinyl acetate is present at a concentration of about 10%, of the film's dry weight. In some embodiments, the polyvinyl acetate is present at a concentration of about 14%, of the film's dry weight. In some embodiments, the polyvinyl acetate is present at a concentration of about 18%, of the film's dry weight.

Some embodiments provide a film wherein the one or more cellulosic polymers are present at a concentration of from about 15 to about 30%, of the film's dry weight. Other embodiments provide a film wherein the one or more cellulosic polymers are present at a concentration of from about 18 to about 22%, of the film's dry weight.

Further embodiments provide a film wherein the metal particles are present at a concentration of greater than about 25% of the film's dry weight. Still further embodiments provide a film wherein the metal particles are present at a concentration of from about 30 to about 60% of the film's dry weight. Yet other embodiments provide a film wherein the metal particles are present at a concentration of from about 45 to about 55% of the film's dry weight.

In some embodiments, the metal particles are zinc particles. In other embodiments, the zinc particles are zinc oxide particles.

In some embodiments, at least one of the one or more cellulosic polymers is hydroxypropyl methyl cellulose.

Some embodiments provide an aqueous oral care composition comprising: any one of the films described herein; and an orally acceptable aqueous carrier.

In some embodiments, the composition has a G' to G" ratio of greater than or equal to 1. In some embodiments, the one or more of the films described herein are suspended in the aqueous carrier.

In some embodiments, the presence of the film does not increase the optical density of any one of the compositions described herein at 610 nm by more that 0.01 after 20 hours compared to the optical density at 610 nm of the composition free of the film after 20 hours. In other embodiments, the film does not increase the optical density of any one of the compositions described herein at 610 nm by more that 0.002 after 20 hours compared to the optical density at 610 nm of the composition free of the film after 20 hours.

In some embodiments, the film is present at a concentration of from about 0.01 to about 5%, by weight of the composition. In some embodiments, the film is present at a concentration of from about 0.1 to about 2%, by weight of the composition. In some embodiments, the film is present at a concentration of from about 0.5 to about 1%, by weight of the composition. In some embodiments, the film is present at a concentration of about 0.5%, by weight of the composition. In some embodiments, the film is present at a concentration of about 1%, by weight of the composition.

In some embodiments, the composition reduces the amount of volatile sulfur compounds in the oral cavity by at least 25%. In some embodiments, the composition reduces the amount of volatile sulfur compounds in the oral cavity by at least 30%. In some embodiments, the composition reduces the amount of volatile sulfur compounds in the oral cavity by at least 35%. In some embodiments, the composition reduces the amount of volatile sulfur compounds in the oral cavity by at least 40%. In some embodiments, the composition reduces the amount of volatile sulfur compounds in the oral cavity by at least 45%. In some embodiments, the composition reduces the amount of volatile sulfur compounds in the oral cavity by at least 50%. In some embodiments, the composition reduces the amount of volatile sulfur compounds in the oral cavity by at least 55%.

Disclosed herein is a method of treating or preventing a disease or condition of the oral cavity comprising contacting an oral cavity surface of a subject in need thereof, with any one of the compositions described herein. In some embodiments, the disease or condition of the oral cavity is selected from gingivitis; periodontitis; and halitosis.

Also provided is a method of reducing volatile sulfur compounds in the oral cavity of subject in need thereof, comprising contacting an oral cavity surface with any one of the compositions described herein.

Methods of making the films comprise the steps of forming a slurry comprising one or more cellulosic polymers, polyvinyl acetate and metal particles, dispensing the slurry on a surface wherein the slurry forms a layer of slurry on the surface, and drying the layer of slurry to remove solvent and produce a polymer matrix film. The resultant films may be included in fluid compositions such as mouthwashes and oral rinses.

In some embodiments, the water-stable film may further comprise additives such as, for example, colorants, flavorants, sweeteners, breath fresheners, whitening agents, and/or therapeutic agents such as agents that promote oral health, e.g. healthy teeth, gums and other oral tissue, and agents that prevent and treat various oral maladies. In addition, the water-stable film may include other film forming agents, plasticizing agents, surfactants and emulsifying agents. The water-stable film may be cut or otherwise divided into multiple pieces such as flakes or small strips and added to a composition where they may provide aesthetic elements and/or serve as a carrier for one or more additives which may be included.

Examples of suitable cellulose derivatives include, but are not limited to: hydroxyalkyl methyl celluloses such as hydroxypropyl methyl cellulose, hydroxybutyl methyl cellulose, hydroxyethyl methyl cellulose, hydroxymethyl methyl cellulose and hydroxyethylpropyl methyl cellulose; carboxyalkyl methylcelluloses such as carboxypropyl methyl cellulose, carboxybutyl methyl cellulose, carboxyethyl methyl cellulose, carboxymethyl methyl cellulose and carboxyethylpropyl methyl cellulose; hydroxyalkyl celluloses such as hydroxypropyl cellulose, hydroxybutyl cellulose, hydroxyethyl cellulose, hydroxymethyl cellulose and hydroxyethylpropyl cellulose; alkyl celluloses such as propyl cellulose, butyl cellulose, ethyl cellulose, methyl cellulose; and carboxyalkyl celluloses such as carboxypropyl cellulose, carboxybutyl cellulose, carboxyethyl cellulose, carboxymethyl cellulose and carboxyethylpropyl cellulose.

Cellulose and cellulose ether derivative polymers may be of any length or combination of lengths. Moreover, the ranges of percent of substitutions may vary to ranges up to about 100%. In molecules comprising two or more different substituting groups, the percentage substitution for each group is independent of the other groups.

Water stable films may comprise a single polymer type of cellulose or cellulose ether derivative, or may comprise a combination of one or more of cellulose and cellulose ether derivatives.

In some embodiments, the film forming agent used to prepare the water-stable films includes a hydroxyalkyl methyl cellulose. In some embodiments, the hydroxyalkyl methyl cellulose used is hydroxypropyl methyl cellulose (HPMC). HPMC is available commercially from the Dow Chemical Company under the trade designation Methocel. Methocel is provided in various forms including Methocel E5 and Methocel E50. In some embodiments, the Methocel used is a combination of Methocel E5 and Methocel E50. In some embodiments, the combination of Methocel E5 and Methocel E50 is provided in a ratio of about 1:1.

Polyvinyl acetate (PVA) is comprised of polymerized vinyl acetate monomers. The degree of polymerization of PVA is typically 100 to 5000. Compositions comprising PVA are sold by BASF under the tradename Kollicoat which contains about 27% PVA. Copolymers containing mixtures of vinylpyrollidone (VP) and vinylacetate (VA) are also suitable for this use. Increasing the level of VP relative to VA in the co-polymer makes the film less stable in water, but also reduces the need for another film-forming polymer, such as the cellulosic polymers described above.

PVA modifies the characteristic of the polymer matrix film in high water systems. A balance between the HPMC and PVA polymers is provided to allow the film to act as a delivery system where it entraps the active ingredient in the mouthwash and delivers it during use. In some embodiments, the ratio of PVA concentration in the film's dry weight to total water content of the composition is from about 1:3 to about 1:7. In some embodiments, the ratio of PVA concentration in the film's dry weight to total water content of the composition is about 1:5. In some embodiments, the ratio of PVA concentration in the film's dry weight to total water content of the composition is about 1:6.

In some embodiments, the metal particles are zinc particles. Representative metal particles suitable for use in the compositions described herein include silicon oxide (SiO₂), molybdenum oxide (Mo₂O₃), aluminum oxide (Al₂O₃), titanium oxide (TiO), zirconium oxide (ZrO₂) and zinc oxide (ZnO).

Particle size may be from about 1 to about 1000 nm. Preferably the particles have an average particle size of about 1 µm to about 850 nm, about 50 µm to about 150 nm, about 15 nm to about 500 nm, about 30 nm to about 250 nm and/or about 5 µm to about 100 nm.

In some embodiments, the particles are non-aggregated. By non-aggregated it is meant that the particles are not massed into a cluster having a size greater than about 1 micron, preferably not greater than about 950 nm or 850 nm. However, particles may be mixed with aggregated particles and other metal particles that have an average particle size of greater than 1 micron if desired. In some embodiments, more than 80% of the particles are non-aggregated. In some embodiments, more than 90% of the particles are non-aggregated.

In some embodiments, the metal particles are zinc oxide particles, having an average particle size of from about 5 nm to about 200 nm.

Optionally, a diol may be included in the polymer matrix film. Examples of diols include propylene glycol and ethylene glycol.

Surfactants may optionally be included in the polymer matrix film. In some embodiments, the optional surfactant is a non-ionic surfactant such as polysorbate 80.

Other additives provided herein may be included in the films and such other additives may also be useful additives in a fluid composition, independent of whether or not included in the films. In some embodiments, the additives may be incorporated into the films but not the aqueous carrier, in others the additives may be incorporated into both the films and the aqueous carrier. Moreover, many such additives may be included in the aqueous carrier, but not the films. In the case of multiple additives, each individual may be present in the films or the aqueous carrier or both independent of the presence or location of any others.

In some embodiments, the additives that may be incorporated in the film matrix are compounds that may be reactive with other ingredients and must therefore be isolated from the other ingredients during manufacture and storage.

In some embodiments, the composition further comprises one or more components selected from a fluoride ion source; a tartar control agent; a buffering agent; an abrasive; and a combination of two or more thereof. In some embodiments, at least one of the one or more components is a fluoride ion source selected from: stannous fluoride, sodium fluoride, potassium fluoride, sodium monofluorophosphate, sodium fluorosilicate, ammonium fluorosilicate, amine fluoride, ammonium fluoride, and a combination of two or more thereof.

Some embodiments further comprise a colorant. Colorants used to prepare the films, as well as the aqueous carrier into which the films may be suspended, are pharmacologically and physiologically non-toxic when used in the suggested amounts. The colorants include both pigments and dyes. Useful pigments include non-toxic, water insoluble inorganic pigments such as titanium dioxide, titanium dioxide coated mica (Timiron), chromium oxide greens, ultramarine blues and pinks and ferric oxides as well as water insoluble dye lakes prepared by extending calcium or aluminum salts of FD&C dyes on alumina such as FD&C Green #1 lake, FD&C Blue #2 lake, FD&C R&D #30 lake and FD&C # Yellow 15 lake. The pigments may have a flake size in the range of 5 to 1000 microns, and in some embodiments, the range may be 250 to 500 microns. Classes of dyes which may be used are available from Micropowders, Inc. under the trade designation Spectra beads which are high molecular weight polyethylene powders permanently colored with dyes such as FD&C Blue #1 aluminum lake.

Dyes, which may be distributed uniformly throughout the film matrix, may be food color additives presently certified under the Food Drug & Cosmetic Act for use in food and ingested drugs, including dyes such as FD&C Red No. 3 (sodium salt of tetraiodofluorescein), Food Red 17, disodium salt of 6-hydroxy-5-{(2-methoxy-5-methyl-4-sulphophenyl)azo}-2-n- aphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-.DELTA.-3,5-cyclohexadienimine], FD&C Blue No. 1 (disodium salt of dibenzyldiethyl-diamino-triphenylcarbinol trisulfonic acid anhydrite), FD&C Blue No. 2 (sodium salt of disulfonic acid of indigotin) and mixtures thereof in various proportions.

Flavoring agents incorporated in the film matrix are known, such as natural and artificial flavors. These flavoring agents may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavoring agents can be used individually or in admixture. Commonly used flavoring agents include mints such as peppermint, artificial vanilla, cinnamon derivatives, and various fruit flavors, whether employed individually or in admixture. Generally, any flavoring agent or food additive, such as those described in Chemicals Used in Food Processing, publication 1274 by the National Academy of Sciences, pages 63-258, may be used.

Sweeteners suitable for use in the compositions described herein include both natural and artificial sweeteners. Suitable sweeteners include water soluble sweetening agents such as monosaccharides, disaccharides and polysaccharides such as xylose, ribose, glucose (dextrose), mannose, galactose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame). In general, the effective amount of sweetener is utilized to provide the level of sweetness desired for a particular film matrix composition, will vary with the sweetener selected. This amount will normally be about 0.01% to about 2% by weight of the composition.

Whitening agents, material which is effective to effect whitening of a tooth surface to which it is applied, such as hydrogen peroxide and urea peroxide, high cleaning silica, silicones, and chlorophyll compounds may be provided in the film. In various embodiments, the compositions of this invention comprise a peroxide whitening agent, comprising a peroxide compound. A peroxide compound is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof.

In various embodiments, the peroxide compound comprises hydrogen peroxide, urea peroxide, sodium percarbonate and mixtures thereof. In some embodiments, the peroxide compound comprises hydrogen peroxide. In some embodiments, the peroxide compound consists essentially of hydrogen peroxide. In some embodiments a non-peroxide whitening agent may be provided. Whitening agents among those useful herein include non-peroxy compounds, such as chlorine dioxide, chlorites and hypochlorites. Chlorites and hypochlorites include those of alkali and alkaline earth metals such as lithium, potassium, sodium, magnesium, calcium and barium. Non-peroxide whitening agents also include colorants, such as titanium dioxide and hydroxyapatite. One or more whitening agents are optionally present in a tooth-whitening effective total amount.

Optional breath freshening agents may be provided. Any orally acceptable breath freshening agent can be used, including without limitation zinc salts such as zinc gluconate, zinc citrate and zinc chlorite, alpha-ionone and mixtures thereof. One or more breath freshening agents are optionally present in a breath freshening effective total amount.

Optionally, an abrasive may be provided as a polishing agent. Any orally acceptable abrasive can be used, but type, fineness (particle size) and amount of abrasive should be selected so that tooth enamel is not excessively abraded in normal use of the composition. Suitable abrasives include silica, for example in the form of silica gel, hydrated silica or precipitated silica, alumina, insoluble phosphates, calcium carbonate, resinous abrasives such as urea-formaldehyde condensation products and mixtures thereof. Among insoluble phosphates useful as abrasives are orthophosphates, polymetaphosphates and pyrophosphates. Illustrative examples are dicalcium orthophosphate dihydrate, calcium pyrophosphate, beta-calcium pyrophosphate, tricalcium phosphate, calcium polymetaphosphate and insoluble sodium polymetaphosphate. Average particle size of an abrasive, if present, is generally about 0.1 to about 30 µm, for example about 1 to about 20 µm or about 5 to about 15 µm.

In embodiments in which prophylactic and therapeutic agents that are incorporated in the film matrix are compounds that are reactive with other ingredients, the prophylactic and therapeutic agents entrained in the film are maintained substantially separate from the ingredients of the carrier during manufacture and storage, and are released during use of the composition. Entrainment of the prophylactic and therapeutic agents in the film prevents premature leakage into the carrier so that in the case of prophylactic and therapeutic agents which are reactive ingredients, interaction with other ingredients is avoided.

For example, reaction of a cationic prophylactic and therapeutic agents such as cetyl pyridinium chloride or chlorhexidene with an anionic surfactant such as sodium lauryl sulfate, which surfactant is conventionally included in oral care compositions, inactivates the therapeutic agent thereby reducing the antibacterial efficacy of the oral care composition.

Optionally, the films may comprise a stannous ion source useful, for example, as a periodontal active, tartar control agent, anticaries agent or tooth desensitizer. Any orally acceptable stannous ion source can be used, including stannous fluoride, other stannous halides such as stannous chloride dihydrate, organic stannous carboxylate salts such as stannous formate, acetate, gluconate, lactate, tartrate, oxalate, malonate and citrate, stannous ethylene glyoxide and the like.

Optionally, the films may include a tartar control (anticalculus) agent. Tartar control agents among those useful herein include phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof, wherein sodium can optionally be replaced by potassium or ammonium. Other useful anticalculus agents include polycarboxylate polymers and polyvinyl methyl ether/maleic anhydride (PVME/MA) copolymers, such as those available under the Gantrez™ brand from ISP, Wayne, N.J.

Other optional additives include antimicrobial (e.g., antibacterial) agents. Any orally acceptable antimicrobial agent can be used, including Triclosan (5-chloro-2-(2,4-dichlorophenoxy)phenol); 8-hydroxyquinoline and salts thereof, zinc and stannous ion sources such as zinc citrate, zinc sulfate, zinc glycinate, sodium zinc citrate and stannous pyrophosphate; copper (II) compounds such as copper (II) chloride, fluoride, sulfate and hydroxide; phthalic acid and salts thereof such as magnesium monopotassium phthalate; sanguinarine; quaternary ammonium compounds, such as alkylpyridinium chlorides (e.g., cetylpyridinium chloride (CPC), combinations of CPC with zinc and/or enzymes, tetradecylpyridinium chloride, and N-tetradecyl-4-ethylpyridinium chloride,); bisguanides, such as chlorhexidine digluconate, hexetidine, octenidine, alexidine; halogenated bisphenolic compounds, such as 2,2' methylenebis-(4-chloro-6-bromophenol); benzalkonium chloride; salicylanilide, domiphen bromide; iodine; sulfonamides; bisbiguanides; phenolics; piperidino derivatives such as delmopinol and octapinol; magnolia extract; grapeseed extract; thymol; eugenol; menthol; geraniol; carvacrol; citral; eucalyptol; catechol; 4-allylcatechol; hexyl resorcinol; and methyl salicylate. A further illustrative list of useful antibacterial agents is provided in U.S. Pat. No. 5,776,435, Gaffar, et al., issued Jul. 7,1998.

Antioxidants are another class of optional additives. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

Also optional, a saliva stimulating agent, useful for example in amelioration of dry mouth may be included. Any orally acceptable saliva stimulating agent can be used, including without limitation food acids such as citric, lactic, malic, succinic, ascorbic, adipic, fumaric, and tartaric acids, and mixtures thereof. One or more saliva stimulating agents are optionally present in a saliva stimulating effective total amount.

Optionally, an antiplaque (e.g., plaque disrupting) agent may be included. Any orally acceptable antiplaque agent can be used, including without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and mixtures thereof.

Optional desensitizing agents include potassium citrate, potassium chloride, potassium tartrate, potassium bicarbonate, potassium oxalate, potassium nitrate, strontium salts, and mixtures thereof. In some embodiments, a local or systemic analgesic such as aspirin, codeine, acetaminophen, sodium salicylate or triethanolamine salicylate can be used.

Optional additives also include nutrients and/or proteins. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, pantheon, retinyl palmitate, tocopherol acetate, and mixtures thereof. Nutritional supplements include amino acids (such as L-tryptophane, L-lysine, methionine, threonine, levocamitine and L-carnitine), lipotropics (such as choline, inositol, betaine, and linoleic acid), fish oil (including components thereof such as omega-3 (N-3) polyunsaturated fatty acids, eicosapentaenoic acid and docosahexaenoic acid), coenzyme Q10, and mixtures thereof.

Herbs such as *Chamomilla recutita, Mentha piperita, Salvia officinalis, Commiphora myrrha* may optionally be included. Suitable proteins include milk proteins and enzymes such as peroxide-producing enzymes, amylase, plaque-disrupting agents such as papain, glucoamylase, glucose oxidase, and "next generation" enzymes."

In preparing the films, the cellulose and/or cellulose derivative(s), PVA and the metal particles and any of the optional ingredients are dissolved in a compatible solvent to form a film forming composition. The film forming composition is cast on a releasable carrier and dried to form a sheet of film matrix material. In some embodiments, the carrier material has a surface tension which allows the film solution to spread evenly across the intended carrier width without soaking to form a destructive bond between the film carrier substrates. Examples of suitable carrier materials include glass, stainless steel, Teflon® and polyethylene-impregnated paper. Drying of the film may be carried out at high temperature using a drying oven, drying terminal, vacuum drier, or any other suitable drying equipment which does not adversely affect the ingredients of which the film is composed.

The film thickness ranges in size from 0.0127 mm to 0.254 mm (0.5 to 10 milli-inch) and preferably 0.0508 mm to 0.0762 mm (2 to 3 milli-inch). The dried film of the present invention is then cut or punched into shaped flakes having a particle size of 5 to 50 mesh, preferably 10 to 20 mesh.

Additional stability can be provided to the shapes formed from the dried film, by applying to the film, before shaping into flakes or small strips, a protective barrier overcoat such as a food grade shellac or ethyl cellulose.

The dried film is then processed for inclusion in the aqueous oral care composition. The film may be cut or punched into small strips or squares. When the film is to be used for decorative effect, the film once formed is punched into various attractive shaped flakes such as hearts, stars, diamonds and circles.

The slurries that are precursors to the films may be characterized using rheology. In some embodiments, the viscoelastic properties of the film slurry, as quantified using G' as an indicator of the structural character of the polymer-particle network, may be about 220-560. In some embodiments G' is about 223-550. In some embodiments, the structure of the polymer-particle matrix is not weak and the slurry is not essentially liquid-like. In some embodiments, the structure of the polymer-particle matrix is not very rigid thereby not leading to the formation of a very brittle film. In some embodiments, the viscosity profile as a function of shear rate is quantified as a measure of flowability and processability the slurries. In some embodiments, the viscosity profiles are not a semi-dilute solution. The viscosity in poise is measured at 0.3 s⁻¹. In some embodiments, the viscosity (taken at 0.3 s⁻¹) for the various slurries is about 175-475. In some embodiments, the viscosity (taken at 0.3 s⁻¹) for the various slurries is about 183-450.

The mechanical properties of the films themselves may also be characterized by, for example, using a Dynamic Mechanical Analyzer to determine the physical stability of various films. The glass transition temperature (Tg) is the temperature at which the film softens. A higher Tg indicates a stronger film. In some embodiments, the Tg (°C) is about -30 to -50 °C. In some embodiments it is about -32 °C. In addition, the storage modulus (E'), which measures the stiffness of the film, indicates the strength of the film. A higher E' correlates to a higher degree of strength. Steric stabilization results from the metal particles restricting polymer motion. In the absence of restriction of polymer motion, films may exhibit cosmetic instability, such as curling. As the polymer network becomes increasingly restricted, the film stiffens. However, excessive restriction can disrupt the polymer structure, resulting in the film becoming brittle and cracked. In some embodiments the E' (MPa) at 1Hz is greater than 1300. In some embodiments the E' (MPa) at 1Hz is about 1400-1900. In some embodiments the E' (MPa) at 1Hz is about 1800.

In some embodiments, the films are rupturable or break down during gargling so that while one or more additives may be maintained substantially separate from other ingredients during manufacture and storage, the one or more additives are subsequently released when the fluid composition is maintained and agitated within the oral cavity. The mechanical agitation created during gargling may effect or facilitate the rupture or breakdown of the film matrix whereby the entrained ingredient is released into the oral cavity.

In some embodiments, the films are retained on the oral mucosa during and after use of the product, from which they slowly erode and release the ingredients contained within the film matrix. The slow release can extend the impact of these ingredients, to improve therapeutic benefit and/or sensory stimulation.

In some embodiments, the composition is an aqueous oral care composition such as a mouthwash or oral rinse, comprising an orally acceptable aqueous carrier. The total amount of water is typically in the range of from about 50% to about 90%, by weight. In some embodiments, the total amount of water is in the range of from about 55% to about 85% by weight. In some embodiments, the total amount of water is in the range of from about 60% to about 80% by weight. Some embodiments are 70% to about 80%, by weight, water. Some embodiments are about 70%, about 71%, about 72%, about 73%, about 74%, about 76% about 77%, about 78%, about 79%, or about 80% water.

In addition to water, other components of the fluid composition may include one or more of humectants, diols, surfactants and active ingredients.

Humectants such as polyol and sugar alcohol solutions may be present in the amount of about 1 to about 25%, each by weight.

Sorbitol and/or another sugar alcohol are generally present, typically in the range of from 0.1 to about 25%, by weight. Some embodiments comprise sorbitol at a concentration of from about 5 to about 15%, by weight. Some embodiments have sorbitol present at about 10%, by weight. Reference herein to sorbitol refers to the material typically available as a 70% aqueous solution.

Glycerin and/or a similar polyol are generally present, typically in the range of from 0 to about 25%, by weight, each. Some embodiments have glycerin present 5-15%. Some embodiments have glycerin present about 7.5%. Another solvent, the diol propylene glycol, may be present. When present, propylene glycol is typically present at a concentration of from about 0.1 to about 50%, by weight. Some embodiments have propylene glycol present from about 5 to about 15%, by weight. Some embodiments have propylene glycol present at a concentration of about 7%, by weight. Some embodiments have propylene glycol present at a concentration of from about 7 to about 12%, by weight. Some embodiments have propylene glycol present at a concentration of from about 9 to about 11%. Polypropylene glycol may be present in an amount equal to about 10%, by weight. Other examples of humectants/polyols include the diol ethylene glycol, and polyols dipropylene glycol and hexylene glycol, cellosolves such as methyl cellosolve and ethyl cellosolve, vegetable oils and waxes containing at least about 12 carbons in a straight chain such as olive oil, castor oil and petrolatum and esters such as amyl acetate, ethyl acetate and benzyl benzoate.

Typically, at least 80% of the aqueous oral care composition is made up of one or more of water, sorbitol, glycerin and propylene glycol. Some embodiments provide aqueous oral care composition comprising at least 90%, water and propylene glycol.

In some embodiments, the composition comprises a preservative. In some embodiments, the preservative is sodium benzoate. In some embodiments, the preservative is present at a concentration of from about 0.01 to 10% wt/wt. In some embodiments, the preservative is present at 0.5, by weight of the composition.

In some embodiments, colorants are present in very small quantities.

A flavoring agent, if included, may be present at a concentration of from about 0.01 to about 1%, by weight. In some embodiments, the flavoring agent may be present at a concentration of about 0.2%, by weight.

Sweeteners would normally be present about 0.001% to about 5% by weight of the composition.

In some embodiments, the sweetener is sodium saccharin and is present at a concentration of about 0.01% by weight of the composition.

Optionally, fluoride salts may be used as anticavities agents. In the use of fluoride salts as anticavities agents, sodium fluoride may be provided at about 0.02%, by weight. Tartar control agents may be present in amounts based upon their activity but generally in the range of 0.01-10%, by weight; in some embodiments, from about 0.1 to about 5%, by weight; in other embodiments, about 1%, by weight.

Sodium phosphate monobasic and/or sodium phosphate monobasic phosphate may be present each at a concentration of from about 0.01 to about 10%, by weight; in some embodiments, from about 0.01 to about 5%, by weight; in other embodiments, about 1%, by weight; and in some embodiments, at about 0.15%, by weight.

In some embodiments, an antimicrobial is present at a concentration of from about 0.001 to about 1%, by weight. In some embodiments, cetylpyridinium chloride is present at a concentration of from about 0.001 to about 1%, by weight; and preferably at a concentration of about 0.05%, by weight.

Any orally acceptable pH modifying agent can be used, including without limitation carboxylic, phosphoric and sulfonic acids, acid salts (e.g., monosodium citrate, disodium citrate, monosodium malate, etc.), alkali metal hydroxides such as sodium hydroxide, carbonates such as sodium carbonate, bicarbonates, sesquicarbonates, borates, silicates, phosphates (e.g., monosodium phosphate, trisodium phosphate, pyrophosphate salts, etc.), imidazole and mixtures thereof. One or more pH modifying agents are optionally present in a total amount effective to maintain the composition in an orally acceptable pH range.

The fluid compositions of the present invention may comprise a blend of polymers which forms a polymer network that is viscoelastic in nature. The fluid compositions exhibit shear thinning behavior when used. Under the stress applied to the liquid in the ordinary use of the mouthwash or oral rinse, the viscosity of the liquid decreases resulting in a more freely flowing solution which provides a wide coverage of the entire oral cavity when used. The viscosity of the fluid compositions is relatively high. Use of the solution results in delivery of a film that coats both the hard and soft tissue of the oral cavity, thereby promoting lubrication, restoring moisture and providing a pleasant mouthfeel. The shear thinning properties of the fluid composition are similar to those of saliva.

The polymer mixture included in the fluid composition may include one or more of xanthan gum, a cellulose derivative cellulose gum, and synthetic high molecular weight polymers of acrylic acid known as carbomer. Cellulose derivative polymers such as cellulose gum may be of any length or combination of lengths. Synthetic high molecular weight polymers of acrylic acid known as carbomer include may be homopolymers of acrylic acid, crosslinked with an allyl ether pentaerythritol, allyl ether of sucrose or allyl ether of propylene. Carbomer has a USP classification of "carbomer homopolymer Type A". Carbomers have the ability to absorb, retain water and swell to many times their original volume. Carbomers codes (910, 934, 940, 941 and 934P) are an indication of molecular weight and the specific components of the polymer.

The combination of polymers in the aqueous oral care compositions imparts upon the product desirable viscoelastic properties. The shear thinning behavior of the aqueous oral care compositions may be quantified as a flow rate index (n). The flow rate index for water equals 1. Against this standard, the flow rate for shear thinning liquids is <1. The flow rate of the fluid compositions described herein is typically between 0.1 and 0.8, preferably between 0.3 and 0.6.

The overall viscosity index, which is referred to as the consistency index (k) of the fluid compositions is typically high, for example about two orders of magnitude greater than water. Accordingly, the flow rate index is typically low, such as n < 1, and the consistency index is typically high, k>10. This combination provides the desirable mouth feel of the products.

In some embodiments, the amount of the three polymers in the fluid composition is from about 0.01 to about 0.5%, by weight of each; that is from about 0.01 to about 0.5%, by weight, xanthan gum; from about 0.01 to about 0.5%, by weight, cellulose gum; and from about 0.01 to about 0.5%, by weight, carbomer. Some embodiments include 0.083%, by weight, xanthan gum; 0.083%, by weight, cellulose gum; and 0.05%, by weight, carbomer.

The polymer mixture included in the aqueous oral care composition may include gellan gum or a combination of gellan gum and xanthan gum. The presence of gellan gum provides a structured liquid which allows the film flakes to remain suspended rather than precipitate to the bottom of the container. A fluid composition comprising a liquid vehicle, gellan gum, and optionally xanthan gum also comprises a sodium salt. Some embodiments comprise any one of the films described herein; xanthan gum; gellan gum; and an orally acceptable aqueous carrier. In some embodiments, gellan gum and xanthan gum are present wherein the total gum concentration is from about 0.05 to about 0.12% by weight; and wherein the weight ratio of xanthan gum to gellan gum in the composition is from about 3:7 to about 1:1. In some embodiments, the gellan gum is present at a concentration of about 0.7%, by weight. In some embodiments, the xanthan gum is present at a concentration of about 0.3%, by weight.

The invention is further described in the following examples. The examples are merely illustrative and do not in any way limit the scope of the invention as described and claimed.

### EXAMPLES

### Example 1

An exemplary water-stable film of the present invention may comprise two different molecular weights of hydroxypropylmethylcellulose (HPMC) specifically, Methocel E5 and E50 from Dow Chemical was modified to increase the stability of the film matrix to support actives in a high water system specifically mouthwash. The polymer used for increased stability was the PVA containing product Kollicoat from BASF.

The formulations of exemplary polymer matrix films of the present invention (Films I - III) and a comparative film (Film X) are described in Table 1 (below).

**Table 1**

| **Polymer Matrix Films** | | | | |
|---|---|---|---|---|
| | **X** | **I** | **II** | **III** |
| **Ingredient** | **Dry Wt %** | | | |
| Methocel E5 | 16.8 | 9.6 | 10.8 | 10.5 |
| Methocel E50 | 15.2 | 9.6 | 10 | 10.5 |
| Zinc oxide | 50.2 | 47.9 | 49.8 | 52.4 |
| PVA | -- | 18 | 13.8 | 10.2 |
| Titanium dioxide | -- | 2.4 | 2.5 | 2.6 |
| Propylene glycol | 13.6 | 9.4 | 9.8 | 10.3 |
| Polysorbate 80 | 0.9 | 3.1 | 3.3 | 3.5 |
| Flavoring agent | 3.3 | -- | -- | -- |

### Example 2

The stability of an exemplary film of the present invention was evaluated and compared with the stability of Film X. Experiments were performed in aqueous systems. Stability of the films was quantified by optical density ("OD") at specific time points.

### Method

Measure aqueous base into beaker and begin mixing with overhead mixer; add polymer matrix films and mix for 1 minute, discontinue mixing, transfer 100 µL aliquot of mouthwash into 96 well plate, record optical density at 610 nm (ignore OD of cells containing solid film pieces). 400 g mouthwash base, 400 rpm mix speed, 0.40 g film.

Tables 2 and 3 (below) provide data generated by the stability experiments conducted on an exemplary film of the present invention and a comparative film, at specific time points.

**Table 2**

| | MW Base (initial) | MW Base + Film X (initial) | MW Base + Film I (initial) | MW Base (20 hr) | MW Base + Film X (20 hr) | MW Base + Film I (initial) | Empty Cells |
|---|---|---|---|---|---|---|---|
| A | 0.038 | 0.085 | 0.041 | 0.04 | 0.273 | 0.042 | 0.044 |
| B | 0.039 | 0.093 | 0.041 | 0.039 | 0.263 | 0.045 | 0.043 |
| C | 0.039 | 0.102 | 0.043 | 0.058 | 0.255 | 0.045 | 0.043 |
| D | 0.039 | 0.101 | 0.044 | 0.039 | 0.264 | 0.043 | 0.043 |
| E | 0.041 | 0.101 | 0.042 | 0.048 | 0.319 | 0.043 | 0.044 |
| F | 0.042 | 0.105 | 0.043 | 0.039 | 0.233 | 0.043 | 0.045 |
| G | 0.038 | 0.113 | 0.043 | 0.038 | 0.289 | 0.044 | 0.044 |
| H | 0.039 | 0.133 | 0.046 | 0.039 | 0.288 | 0.046 | 0.044 |

The average OD of empty cells was 0.044021. When the OD of the mouthwash was calculated as described in Table 3 (below), the OD reading of a cell containing mouthwash was determined by subtracting 0.044021 from the original reading.

**Table 3**

| **Initial** | **MW Base*** | **Film X** | **Film I** |
|---|---|---|---|
| Avg | -0.00465 ± 0.000498 | 0.060104 ± 0.005048 | -0.00115 ± 0.000581 |

| **20 hr** | **MW Base** | **Film X** | **Film I** |
|---|---|---|---|
| Avg | -0.00152 ± 0.002486 | 0.228979 ± 0.009163 | -0.00015 ± 0.000479 |

In addition to the enhanced film stability illustrated by the above optical density data, compositions comprising 0.5% and 1%, by weight of the films of the present invention, reduced the amount of volatile sulfur compounds in the oral cavity by 42.6% and 57.4%, respectively.

### Example 3

Table 4 below provides data generated by the stability experiments conducted on exemplary films of the present invention, comprising 10% and 14%, by weight, PVA.

**Table 4**

| **% PVA*** | **% Water**** | **Optical Density** |
|---|---|---|
| 10 | 60 | 0.001 |
| 10 | 60 | 0.002 |
| 10 | 60 | 0.002 |
| 10 | 70 | 0.015 |
| 10 | 80 | 0.043 |
| 10 | 80 | 0.033 |
| 10 | 80 | 0.057 |
| 14 | 60 | 0.003 |
| 14 | 65 | 0.003 |
| 14 | 70 | 0.008 |
| 14 | 80 | 0.055 |
| 14 | 80 | 0.030 |

| | | |
|---|---|---|
| * PVA measured as percentage of dry film weight. ** Water concentration represents total water content. | | |

The data described in the Examples section demonstrates that the films of the present invention are stable in aqueous mediums, and at the same time, are able to deliver an active ingredient (e.g. zinc oxide) to the oral cavity during use of the aqueous compositions comprising the films.

## Claims

1. A water-stable film, comprising:
one or more cellulosic polymers present in an amount from 10 to 50% of the film's dry weight,
polyvinyl acetate present in an amount from 10 to 20% of the film's dry weight; and
particles selected from silicon oxide, molybdenum oxide, aluminum oxide, titanium oxide, zirconium oxide and zinc oxide, wherein the particles are present at a concentration of from 30% to 60% of the film's dry weight.

2. The film of claim 1, wherein the one or more cellulosic polymers are present at a concentration of from 15 to 30%, of the film's dry weight.

3. The film claim 1 or claim 2, wherein the one or more cellulosic polymers are present at a concentration of from 18 to 22%, of the film's dry weight.

4. The film of any preceding claim, wherein the particles are present at a concentration of from 45 to 55% of the film's dry weight.

5. The film of any preceding claim, wherein the particles are zinc oxide particles.

6. The film of any preceding claim, wherein at least one of the one or more cellulosic polymers is hydroxypropyl methyl cellulose.

7. An aqueous oral care composition comprising:
the film of any preceding claim; and
an orally acceptable aqueous carrier;
wherein the film is present at a concentration of from 0.1 to 2%, by weight of the composition.

8. The composition of claim 7, wherein the composition has a G' to G" ratio of greater than or equal to 1.

9. The composition of claim 7 or claim 8, wherein the film is suspended in the aqueous carrier.

10. A composition according to any one of claims 7 to 9, for use in the treatment or prevention of gingivitis, periodontitis or halitosis .

## Patentansprüche

1. Wasserbeständiger Film, der Folgendes umfasst:
ein oder mehrere Cellulosepolymere, die in einer Menge von 10 bis 50 % der Trockenmasse des Films vorliegen,
Polyvinylacetat, das in einer Menge von 10 bis 20 % der Trockenmasse des Films vorliegt; und
Partikel, die aus Siliciumoxid, Molybdänoxid, Aluminiumoxid, Titanoxid, Zirconiumoxid und Zinkoxid ausgewählt sind, wobei die Partikel in einer Konzentration von 30 % bis 60 % der Trockenmasse des Films vorliegen.

2. Film nach Anspruch 1, wobei das eine oder die mehreren Cellulosepolymere in einer Konzentration von 15 bis 30 % der Trockenmasse des Films vorliegen.

3. Film nach Anspruch 1 oder 2, wobei das eine oder die mehreren Cellulosepolymere in einer Konzentration von 18 bis 22 % der Trockenmasse des Films vorliegen.

4. Film nach einem vorhergehenden Anspruch, wobei die Partikel in einer Konzentration von 45 bis 55 % der Trockenmasse des Films vorliegen.

5. Film nach einem vorhergehenden Anspruch, wobei die Partikel Zinkoxidpartikel sind.

6. Film nach einem vorhergehenden Anspruch, wobei mindestens eines des einen oder der mehreren Cellulosepolymere Hydroxypropylmethylcellulose ist.

7. Wässrige Mundpflegezusammensetzung, die Folgendes umfasst:
den Film nach einem vorhergehenden Anspruch und
einen oral verträglichen wässrigen Trägerstoff;
wobei der Film in einer Konzentration von 0,1 bis 2 Gew.-% der Zusammensetzung vorliegt.

8. Zusammensetzung nach Anspruch 7, wobei die Zusammensetzung ein Verhältnis von G' zu G " größer gleich 1 hat.

9. Zusammensetzung nach Anspruch 7 oder 8, wobei der Film in dem wässrigen Trägerstoff suspendiert ist.

10. Zusammensetzung nach einem der Ansprüche 7 bis 9 zur Verwendung bei der Behandlung oder Prävention von Gingivitis, Parodontitis oder Halitosis.

## Revendications

1. Un film stable à l'eau, comprenant :
un ou plusieurs polymères cellulosiques présents dans une quantité comprise entre 10 et 50 % du poids sec du film,
de l'acétate de polyvinyle présent dans une quantité comprise entre 10 et 20% du poids sec du film ; et
des particules sélectionnées parmi un oxyde de silicium, un oxyde de molybdène, un oxyde d'aluminium, un oxyde de titane, un oxyde de zirconium et un oxyde de zinc, dans lequel les particules sont présentes à une concentration comprise entre 30% et 60% du poids sec du film.

2. Le film selon la revendication 1, dans lequel le un ou plusieurs polymères cellulosiques sont présents à une concentration comprise entre 15 et 30% du poids sec du film.

3. Le film selon la revendication 1 ou la revendication 2, dans lequel le un ou plusieurs des polymères cellulosiques sont présents à une concentration comprise entre 18 et 22%, du poids sec du film.

4. Le film selon l'une quelconque des revendications précédentes, dans lequel les particules sont présentes à une concentration comprise entre 45 et 55% du poids sec du film.

5. Le film selon l'une quelconque des revendications précédentes, dans lequel les particules sont des particules d'oxyde de zinc.

6. Le film selon l'une quelconque des revendications précédentes, dans lequel au moins un du un ou plusieurs polymères cellulosiques est de l'hydroxypropyl méthyl cellulose.

7. Une composition aqueuse pour soins buccaux comprenant :
le film selon l'une quelconque des revendications précédentes ; et
un véhicule aqueux buccalement acceptable ;
dans lequel le film est présent à une concentration comprise entre 0,1 et 2%, du poids de la composition.

8. La composition selon la revendication 7, dans laquelle la composition a un rapport de G' à G" supérieur ou égal à 1.

9. La composition selon la revendication 7 ou la revendication 8, dans laquelle le film est en suspension dans le véhicule aqueux.

10. Une composition selon l'une quelconque des revendications 7 et 9, à utiliser dans le traitement ou la prévention de la gingivite, la parodontite ou l'halitose.
